(19) Europäisches Patentamt

European Patent Office

Office européen des brevets

(11) **EP 2 038 627 B1**

(12) **EUROPÄISCHE PATENTSCHRIFT**

(45) Veröffentlichungstag und Bekanntmachung des Hinweises auf die Patenterteilung:
**29.03.2017 Patentblatt 2017/13**

(21) Anmeldenummer: **07801133.5**

(22) Anmeldetag: **29.06.2007**

(51) Int Cl.:
***G01L 5/16*** *(2006.01)*

(86) Internationale Anmeldenummer:
**PCT/DE2007/001153**

(87) Internationale Veröffentlichungsnummer:
**WO 2008/000246 (03.01.2008 Gazette 2008/01)**

(54) **KRAFTSENSOR UND VERFAHREN ZUM ERFASSEN MINDESTENS EINER KRAFTKOMPONENTE**

FORCE SENSOR AND METHOD FOR DETECTING AT LEAST ONE FORCE COMPONENT

DETECTEUR DE FORCE ET PROCEDE SERVANT A DETECTER AU MOINS UNE COMPOSANTE DE FORCE

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **29.06.2006 DE 102006030407**

(43) Veröffentlichungstag der Anmeldung:
**25.03.2009 Patentblatt 2009/13**

(73) Patentinhaber: **Technische Universität Darmstadt 64285 Darmstadt (DE)**

(72) Erfinder:
• **MEISS, Thorsten**
  **64285 Darmstadt (DE)**
• **KERN, Thorsten**
  **64342 Seeheim-Jugenheim (DE)**
• **KLAGES, Stephanie**
  **64807 Dieburg (DE)**
• **WERTHSCHÜTZKY, Roland**
  **14532 Kleinmachnow (DE)**

(74) Vertreter: **Dosterschill, Peter et al
Dosterschill & Kollegen
Fichtenstrasse 11
85570 Ottenhofen (DE)**

(56) Entgegenhaltungen:
**DE-A1- 3 940 696     US-B1- 6 221 023**

**Beschreibung**

Die Erfindung betrifft einen Kraftsensor zur bevorzugten Anwendung an der Spitze einer lang gestreckten Einrichtung gemäß Oberbegriff des Anspruchs 1 sowie ein Verfahren zur Messung einer Kraft und deren Wirkrichtung gemäß Oberbegriff des Anspruchs 13.

Ein besonderer Anwendungsfall dieser Erfindung betrifft die Kathetertechnik, die von einer lang gestreckten Einrichtung zum zumindest teilweisen Einführung in einen Organismus durch eine Körperöffnung bestimmt ist. Diese lang gestreckten Einrichtungen kommen vor allem in der minimal invasiven Chirurgie und Diagnostik an insbesondere menschlichen Körpern zum Einsatz. Damit bei der Invasion der lang gestreckten Einrichtung keine Körpergefäße durch körpernahe Spitze der von dem behandelnden Arzt in der Regel manuell zu bedienenden lang gestreckten Einrichtung verletzt werden, orientiert sich der Arzt notwendigerweise daran, welche Kräfte ihm an einer Handhabe der lang gestreckten Einrichtung mitgeteilt werden. Wegen der Reibung und der eingeschobenen, im Verlauf der Invasion des Katheters oder Führungsdrahtes in den Körper kontinuierlich zunehmenden Katheter-/Führungsdrahtmasse, gibt die dem behandelnden Arzt an der Handhabe mitgeteilte Kraft einen kaum noch nutzbaren Aufschluss über die tatsächlich an der Katheter-/Führungsdrahtspitze auftretenden Kräfte. Damit der behandelnde Arzt der Handhabe des Katheters die richtige Betätigungskraft mitteilen kann, ist ein außergewöhnlich reicher Erfahrungsschatz bei der Bedienung von Katheter-/Führungsdrähten erforderlich.

Aus der DE3940696 A1 ist ein Sensor zum Messen einer Kraft bekannt, wobei der Sensorkörper zumindest in seinem Wirkbereich aus einem ätzbaren Material besteht und eine faltenartige Struktur aufweist.

Aus der DE 103 03 270 A1 ist eine Katheteranordnung bekannt, bei der die auf die Katheterspitze beim Einschieben wirkende Kraft gemessen wird. Die entsprechende Kraft wird dem Arzt über eine haptische Handhabe taktil mitgeteilt. Auf diese Weise wird das Auffinden beispielsweise von Aderabzweigen oder Perforationen an der Herzscheidewand, gerade für einen unerfahrenen Arzt erleichtert und bildet die Basis für eine intuitive Handhabung der Einrichtung. Eine die Kraft an der Spitze repräsentierende Messgröße verwendende, elektrodynamische Antriebsvorrichtung zur Erzeugung der haptischen Kraftvorspannung ist aus der DE 103 19 081 bekannt. Die hier vorgestellte Er-findung ermöglicht die Kraftmessung an der Spitze der lang gestreckten Einrichtung und die Auswertung der Messsignale, was zur Umsetzung der Erfindung aus der DE 103 03 270 A1 erforderlich ist.

Gemäß der US 6,221,023 B1 wird ein Kraftsensor an der Spitze von Kathetern vorgesehen, der auf einem resistiven Funktionsbetrieb basiert. Die in den Sensor eingeleitete Kraft wird durch eine Widerstandsbrückenschaltung aufgenommen. Der Aufbau dieses Sensors ist aufgrund der großen Teilezahl aufwendig. Die damit verbundenen Fertigungs- und Montagekosten machen den bekannten Sensor insbesondere für Katheter aufgrund deren bevorzugten Einweg-Eigenschaft ungeeignet. Weiterhin ist die Fläche zur Primärkontaktierung des Messelementes senkrecht zur Längsrichtung des Katheters ausgeführt und somit die Fläche zur Kontaktierung durch den Durchmesser der lang gestreckten Einrichtung begrenzt. Deshalb, und wegen der hohen Teileanzahl ist die Miniaturisierbarkeit des Kraftsensors insbesondere unterhalb eines Katheterdurchmessers von weniger als 3 mm nur mit einem äußerst hohen konstruktiven Aufwand realisierbar. Aufgrund der großen Abmessungen ist zu schließen, dass der Sensor den Katheterschlauch an der Spitze vollständig verschließt. Damit ist aber die Funktion des Katheterschlauchs, durch den Instrumente und Flüssigkeiten in den Körper eingebracht werden, nicht mehr sinnvoll gegeben. Eine Integration des Sensors in den bei Katheterisierungen benötigten, wesentlich dünneren Führungsdraht ist aufgrund der großen Abmessungen, der hohen Teileanzahl und der ungünstigen Drahtführung durch die senkrecht zur Längsrichtung orientierten Kontaktfläche nicht möglich.

In der JP 06190050 A wird ein taktiler Sensor beschrieben, der an der Außenwandung von Kathetern angebracht werden kann. Es wird vorgeschlagen, diesen Sensor aus einer sehr dünnen Siliziumscheibe herzustellen. Sehr dünne Elemente können aber die auftretenden Kräfte von ca. 300 mN nicht aufnehmen. Die Verwendung dickerer, stabilerer Siliziumscheiben scheidet aus, da eine Biegung dickerer Scheiben, was zur Integration in die Einrichtung notwendig ist, zum Zerbrechen des Material führen würde.

In "Beccai L et al.: Silicon-based three axial Force Sensor for Prothetic Applications. Sensors and Microsystems, Proccedings of the 7th Italien Conference 2002" wird ein Kraftsensor vorgestellt, der in Beinprothesen integriert werden soll. Der Kraftvektor kann bestimmt werden, indem die Kraft in Richtung und Amplitude gemessen wird. Zur Messung der Kräfte werden resistive Elemente eingesetzt, die nicht zu einer "Wheatstone-Brücke" verschaltet sind. Zur Integration in eine lang gestreckte Einrichtung sind aber Brückenverschaltungen anzustreben, wodurch eine hohe Messgenauigkeit erzielt werden kann, da dann die Signale nahezu unabhängig von Zuleitungswiderständen der langen, dünnen Zuleitungen übertragen werden können. Gerade bei in den Körper eingeführten langen Einrichtungen ist sonst das Messsignal abhängig von der Temperatur der Zuleitung und somit von der Einschublänge der Einrichtung. Die Herstellung der Elemente erfordert einen teuren, beidseitigen, mehrstufigen Trockenätzprozess. Trotzdem muss dass Messelement noch über einen speziellen Gegenkörper gelagert werden, um eine Auslenkung der Messbalken zu ermöglichen. Der Sensor besteht somit aus mindestens zwei Teilen, die exakt miteinander verbunden sein müssen. Der freigelegte Kraftaufnehmer, genannt Mesa, weist konstruktionsbedingt eine große Länge, nämlich ungefähr eine Länge, die der Dicke des Ausgangsmaterials entspricht, auf. Somit zeigt dieser Sensor prinzipbedingt immer eine um ein Vielfaches höhere Messempfindlichkeit, aber auch Anfälligkeit gegen Zerstörung, gegenüber seitlichen Kräften, im Vergleich zu der in Längsrichtung wirkenden Kraft. Das ist ungünstig, da es wünschenswert ist, besonders die Kräfte in Längsrichtung der Einrichtung zu messen bzw. eine ausgewogene Empfindlichkeit für unterschiedliche Kraftkomponenten zu erzielen. Das Messelement weist eine zur Längsrichtung senkrechte Ebene der elektrischen Kontakte auf, so dass die Kontaktierungsfläche auf den Durchmesser der Einbaufläche begrenzt ist, was eine Kontaktierung erschwert. Das Messelement weist einen gegenüber Führungsdrähten großen Durchmesser von etwa 1 mm auf, so dass eine Integration in den Führungsdraht nicht möglich ist.

Dies zeigt, dass der Stand der Technik von taktilen Sensoren zur Integration in Katheter nicht den gestellten Anforderungen bezüglich Miniaturisierung, hoher Stabilität, einfacher Herstellung und niedriger Kosten entspricht.

Es ist **Aufgabe** der Erfindung, einen Kraftsensor oben genannter Gattung anzugeben, der einfach aufgebaut ist und aus einem Teil besteht, der in lang gestreckte Einrichtungen mit einem Durchmesser von weniger als 3 mm, insbesondere 0,33 mm Durchmesser (1 French), integrierbar ist und Kräfte erfassen kann, welche zumindest teilweise an der lang gestreckten Einrichtung in Längsrichtung angreifen. Weiterhin soll dem Anwender ein kraftabhängiges Drehmoment vermittelt werden, mit Erfassung des an der Führungsdrahtspitze anliegenden Kraftvektors, also der Bestimmung des Kraftbetrages in drei unabhängigen Richtungen, vorzugsweise auch Rückschluss auf die Richtung der angreifenden Kraft ermöglicht und so Kräfte nach Betrag und Richtung bestimmt werden können.

Diese Aufgabe wird durch einen Kraftsensor mit den Merkmalen des Patentanspruchs 1 gelöst. Vorteilhafte Ausgestaltungen sind in den Unteransprüchen gekennzeichnet.

Dem gemäß ist der erfindungsgemäße Sensor ausgelegt, eine Kraft zu erfassen, die an der lang gestreckten Einrichtung, insbesondere einer lang gestreckten medizintechnischen Einrichtung, wie einem Katheter oder Führungsdraht, angreift, welche Kraft ein nicht zu vernachlässigende Kraftkomponente in Längsrichtung der lang gestreckten Einrichtung aufweisen kann. Der erfindungsgemäße Sensor hat einen Kraftaufnehmer, an dem zumindest der wesentliche Teil der zu erfassenden Kraft entweder über die lang gestreckte Einrichtung oder direkt in den Sensor gerichtet einleitbar ist. Der Sensor soll erfindungsgemäß derart beschaffen sein, dass er an der lang gestreckten Einrichtung anbringbar und insbesondere bei bereits existierenden lang gestreckten Einrichtungen nachrüstbar ist. Erfindungsgemäß weist der Sensor mindestens eine Stirnfläche auf, die im Verhältnis zur Querschnittsfläche des Sensors eine nicht zu vernachlässigende Flächengröße aufweist und die Einkopplung insbesondere der Kräfte mit einer Kraftkomponente in Längsrichtung der lang gestreckten Einrichtung in besonderer Weise ermöglicht. Die einfache Miniaturisierung der Kraftsensoren erlaubt weiterhin die Integration der Kraftsensoren sowohl in die Katheterwandung, ohne die distale Katheteröffnung zu verschließen, als auch die besonders wichtige und anspruchsvolle Integration in den Führungsdraht. Durch besondere Konstruktionsmerkmale und die Anwendung einer Methode zur Signalgewinnung lassen sich die Kräfte an der lang gestreckten Einrichtung in Amplitude und darüber hinaus auch in der Wirkrichtung bestimmen.

Der erfindungsgemäße Sensor bietet die folgenden Vorteile gegenüber den genannten bekannten Kraftsensoren:

- Mit dem erfindungsgemäßen Sensor besteht die Möglichkeit, eine Kraftsensorik in lang gestreckte Einrichtungen zu integrieren, welche eine laterale Ausdehnung oder einen Durchmesser von weniger als 3 mm, insbesondere 0,33 mm (1 French) aufweisen:

- der erfindungsgemäße Sensor besteht aus einem einzelnen Teil und ist aufgrund der geringen Teileanzahl und der Fertigungstechnologie im Scheibenverbund für eine Massenproduktion hervorragend geeignet. Die Fertigungskosten sind sehr gering, Montagekosten für das Sensorelement selbst entfallen, eine Montage an der lang gestreckten Einrichtung ist einfach;

- die besondere Gestaltung und die damit verbundene hohe mechanische Steifigkeit ermöglicht eine sehr einfache, dünne, kostengünstige Gehäusung und erfüllt so ohne weiteres die hohen Anforderungen der Hygiene in der Medizintechnik;

- mit dem erfindungsgemäßen Sensor können aufgrund der bevorzugten Nutzung des piezoresistiven Wirkprinzips sehr genaue Kraftbetrags- und Kraftrichtungsmessungen erzielt werden, unter Verwendung einer kostengünstigen externen Auswerteelektronik;

- mit dem erfindungsgemäßen Sensor können aufgrund der Verwendbarkeit von vorzugsweise Silizium als Grundwerkstoff äußerst genaue Messungen erfolgen, so dass bei Kraftbe- und -entlastung das Ausgangssignal in besonderem Maße die anliegende Kraft wiedergibt;

- der erfindungsgemäße Sensor kann mit gegenwärtig verfügbarer Technologie noch weit unter die geforderten lateralen Abmessungen von 0,3 mm miniaturisiert werden;

- auf dem erfindungsgemäßen Sensor ist in einfacher Weise zusätzliche Elektronik monolytisch integrierbar, so dass die Störeinflüsse der Signalübertragung, im besonderen aber auch die Anzahl der notwendigen elektrischen Versorgungsleitungen auf ein Minimum reduziert werden können. Zur Energie- und Signalübertragung können die schon vorhandenen Komponenten der lang gestreckten Einrichtung genutzt werden, wodurch die Kosten der gesamten Einrichtung durch die Sensorintegration nur sehr gering erhöht werden.

Der erfindungsgemäße Sensor kann Kräfte dem Betrag und/oder der Wirkrichtung nach in Echtzeit und insbesondere kontinuierlich erfassen. Insbesondere ist der erfindungsgemäße Sensor dazu ausgelegt, eine Kraft hauptsächlich in Längsrichtung der lang gestreckten Einrichtung zu erfassen.

Der erfindungsgemäße Sensor wird aus einem Grundelement aufgebaut, welches mindestens einen seitlichen Einschnitt aufweist. Aufgrund dieses Einschnittes entsteht eine Asymmetrie des Grundkörpers, so dass bei einer Kraftbelastung in Längsrichtung der lang gestreckten Einrichtung eine Verbiegung des Messelementes erfolgt. Diese Verbiegung erzeugt mechanische Spannungen, die bevorzugt in der Nähe des Einschnittes mittels spannungs- und dehnungsempfindlicher Widerstände erfasst werden. Die Änderung dieser Messwiderstände gibt somit in einem Proportionalverhältnis die anliegende Kraft wieder. Eine in axialer Richtung eingekoppelte Kraft erzeugt in dem Messelement in Bereichen gleicher Dicke einen über die Länge des Messelementes konstanten Spannungszustand, während die mechanischen Spannungen, die aus seitlicher Krafteinkopplung resultieren, stark vom Ort auf dem Messelement abhängig sind. Durch Integration von mindestens einem Widerstand für jede zu messende Kraftkomponente ist es auch möglich, neben der axialen Kraft den vollständigen Kraftvektor zu bestimmen. Hierfür wird eine Methode beschrieben.

Alle Ausführungsformen können durch Mikrofertigungsverfahren, vorzugsweise die der Volumen-Mikromechanik in Silizium gefertigt werden. Dabei wird eine Siliziumscheibe so strukturiert, dass durch ein Einbringen eines Dotierungsstoffes in das Silizium Widerstände erzeugt werden, die ihren Betrag in Abhängigkeit von der mechanischen Spannung ändern. Diese Widerstände sind aufgrund der elektrischen Wirkung zwischen der Siliziumscheibe und der Wirkung des Dotierstoffes nur an den gewünschten Bereichen elektrisch leitend verbunden, ansonsten untereinander aufgrund der gewählten Herstellung durch Ausbildung eines pn-Überganges voneinander isoliert. Aufgrund der einfacheren Herstellung werden diese Widerstände in der Regel auf der Oberseite des Messelementes und nicht in Vertiefungen in der Siliziumscheibe eingebracht, wie es auch im Folgenden angenommen werden soll. Eine Steigerung der Messempfindlichkeit kann bei dem erfindungsgemäßen Sensor aber erreicht werden, wenn die Widerstände auf der Rückseite der Biegeplatte in dem Einschnitt, beispielsweise durch Strukturierung mit Dickschicht-Resist, eingebracht werden. Die Verfahren der Mikrostrukturierung ermöglichen die Herstellung einer äußerst großen Menge an Sensoren zeitgleich auf einer Scheibe (Batch-Verfahren, Fertigung im Nutzen) und ermöglichen so sehr kostengünstige Messelemente. Alle hier genannten Fertigungsverfahren sind etablierter Stand der Technik, z.B. bei der Fertigung von Druck- und Kraftsensoren.

Neben der Ausführung als miniaturisiertes piezoresistives Messelement ist es im Allgemeinen auch möglich, die vorgestellten Messelemente makroskopisch aufzubauen, beispielsweise unter Verwendung von Metall als Grundkörpermaterial, und zur Spannungs- bzw. Dehnungsmessung Dehnungsmessstreifen einzusetzen. Auch ist es möglich anstatt eindotierter Widerstände anders geartete, spannungs- oder dehnungsabhängige Widerstände aufzubringen, beispielsweise unter Verwendung von Dick- oder Dünnschichttechnologie.

Die im Folgenden dargelegten unterschiedlichen Varianten mit monokristallinem Grundkörper aus Silizium stellen für verschiedene Herstellungsverfahren Vorzugsvarianten dar.

Die erste Ausführungsform zeichnet sich dadurch aus, dass die Strukturierung des Einschnittes des Grundkörpers durch die gut verfügbare Technologie des nasschemischen Ätzens von Silizium mittels beispielsweise KOH hergestellt werden kann. Diese Technologie zeichnet sich durch geringe Investitionskosten aus, so dass sie auch von mittelständischen Firmen angewendet werden kann. Die Herstellung kann in der Art erfolgen, wie sie bei Standarddrucksensoren angewendet wird, bei denen die Membrandicke durch die Ätzzeit bestimmt wird. Auch Herstellungsverfahren mit einem elektrochemischen Ätzstopp an einer Epitaxieschicht sind für Kraftmesselemente für sehr kleine Nennkräfte möglich.

In einer weiteren Ausführungsform wird der Einschnitt des Grundkörpers durch Trockenätzen von der Scheibenrückseite erzeugt. Durch das hohe Aspektverhältnis der steilen Ätzkante ist es möglich, die Aussparung sehr nah an der Kopfplatte zur platzieren und somit mit dem Messelement sehr empfindlich axiale Kräfte zu messen, aber eine geringe Empfindlichkeit für seitliche Kräfte $F_x$ und $F_y$ zu erzielen. In dieser Fertigungsvariante ist es auch möglich, durch die Lage der Aussparung die Empfindlichkeit des Messelementes für unterschiedliche Kraftrichtungen einzustellen. Dies bildet die Grundlage für besonders leistungsfähige Sensoren zur dreidimensionalen Kraftmessung. Die Positionierung der Aussparung nahe der Kopfplatte erhöht weiterhin die Steifigkeit des Grundkörpers und verringert so Einflüsse der Gehäusung bei einem Verguss mit Kunststoff und ermöglicht weiterhin besonders dynamische Messungen.

In einer weiteren Ausführungsform wird der Einschnitt des Grundkörpers durch Sägen von der Scheibenrückseite erzeugt. Anstatt Sägen können auch alternative Mikrobearbeitungsverfahren wie Schleif-, Polier-, Erodier- und Bohrverfahren eingesetzt werden. Allerdings ist Sägen für die Herstellung ein sehr günstiges Verfahren, da Sägen in der Regel auch zur Vereinzelung der strukturierten Messelemente aus der Scheibe eingesetzt wird. Die Vereinzelung der Messelemente und das Erzeugen der Messelemente können somit nahezu zeitgleich mit demselben Prozess erfolgen, vorzugsweise von der Scheibenrückseite. Messelemente sind auf diese Weise äußerst einfach und kostengünstig mit nur zwei Maskenschritten, nämlich zur Dotierung und zur Leiterbahnstrukturierung und ohne Ätztechnik herstellbar.

Weiterhin kann eine nahezu senkrechte Kante des Einschnittes erzeugt werden. Deshalb kann der Einschnitt, wie bei Anwendung des Trockenätzens, nah an der Kopfplatte des Grundkörpers eingebracht werden. Dies steigert die Funktionsfähigkeit des Messelementes, da seitliche Kraftkomponenten einen geringeren Einfluss auf das Messergebnis haben. Weiterhin entfallen bei der Strukturierung des Einschnittes mehrere Prozessschritte, da eine Strukturierung einer Ätzmaske auf der Scheibenrückseite entfallen kann. Dies sind im Speziellen mehrere Reinigungsschritte, das Aufbringen und Strukturieren der Ätzmaske, das Ätzen usw.. Das Verfahren kann durch Wafersägen durchgeführt werden. Zur Erhöhung der Stabilität des Messelementes kann nachfolgend ein kurzer chemischer Ätzschritt zur Verringerung von Mikrorissen erfolgen, wozu allerdings keine Maskierung der Rückseite erfolgen muss. Hierbei können auch weiterhin die Außenkanten des Messelementes abgerundet werden und mögliche Toleranzen des Sägeprozesses sehr exakt und einfach ausgeglichen werden. Diese Ausführungsform bietet somit alle Vorteile der vorhergehenden, durch Trockenätzen hergestellten Variante, bei Reduzierung der Prozessschritte und unter Verwendung sehr kostengünstiger Herstellungstechnologien. Die Bearbeitungsreihenfolge der beschriebenen Schritte kann in Anlehnung der verwendeten Apparaturen in bestimmten Grenzen verändert werden

In einer weiteren Ausführungsform wird die Aussparung zur Erzeugung der Asymmetrie nicht nur durch einen geraden Einschnitt auf der Scheibenrückseite erzeugt, sondern beispielsweise durch Bohren oder spezielle Ätzlöcher in der Scheibenrückseite oder in der Scheibenvorderseite. Es entsteht eine höhere Asymmetrie des Messelementes. Dadurch wird der Messeffekt weiter gesteigert.

Eine weitere Ausführungsform entsteht durch die Bearbeitung von in der Druckmesstechnik standardmäßig eingesetzten Messelementen. Bei Drucksensoren wird durch einen Ätzprozess eine Abdünnung des Grundkörpers unter den Messwiderständen erzielt. Diese Messelemente weisen immer eine stabile Randstruktur auf. Durch Entfernen dieser Randstruktur, beispielsweise durch Sägen oder Ätzen, kann dies Struktur eines Druckmesselementes in eine erfindungsgemäße Struktur überführt werden. Schließlich betrifft die Erfindung eine Methode zur Bestimmung des Betrags der auf die lang gestreckte Einrichtung wirkenden Kraft. Weitere Ausführungsformen dieser Methode ermöglichen im Besonderen die Bestimmung der Kraft nach Betrag und Richtung.

Die Sensoren können beispielsweise an der Spitze von Führungsdrähten, aber auch an beliebiger Stelle in Kathetern eingesetzt werden.

Weitere Vorteile, Merkmale und Eigenschaften der Erfindung werden durch die folgende Beschreibung bevorzugter Ausführungsformen der Erfindung anhand der beiliegenden Zeichnungen deutlich. Es zeigen:

Fig. 1a: eine Prinzipskizzenansicht des erfindungsgemäßen Sensors in einer ersten Ausführung mit einem einseitigen Einschnitt, wie er beispielsweise durch anisotropes nasschemisches Ätzen in Silizium hergestellt werden kann;

Fig. 1b: eine Prinzipskizzenansicht wie in Fig. 1 a, mit Verdeutlichung der elektrischen Funktionselemente wie Widerstände, Leiterbahnen und Anschlusskontakte;

Fig. 2: eine Prinzipskizzenansicht des Grundkörpers mit einem einseitigen Einschnitt, wie er beispielsweise durch Trockenätzen von der Scheibenrückseite hergestellt werden kann;

Fig. 3: eine Prinzipskizzenansicht des Grundkörpers mit einem einseitigen Einschnitt, wie er beispielsweise durch Sägen hergestellt werden kann;

Fig. 4: eine Prinzipskizzenansicht des Grundkörpers mit einem mehrseitigen Einschnitt, wie er beispielsweise durch mehrseitiges Ätzen oder Bohren hergestellt werden kann;

Fig. 5: eine Prinzipskizzenansicht des Grundkörpers, wie er aus einem bekannten Drucksensor durch zweiseitiges Abtrennen der Randbereiche hergestellt werden kann;

Fig. 6a: eine CAD-Zeichnung eines konstruierten Sensorelementes;

Fig. 6b: eine CAD-Zeichnung eines konstruierten Sensorelementes, welches an einem Führungsdraht montiert ist und durch Vergießen oder Umspritzen mit einem weichen, biokompatiblen Kunststoff gehäust ist;

Fig. 7: Messergebnisse des Signalausgangs eines Prototyps bei dynamischer, harmonischer Krafteinleitung;

Fig. 8: eine Prinzipskizze eines Schnittes durch einen Katheter mit integrierten Kraftsensoren;

Fig. 9: die Verschaltung der Widerstände, speziell die Ausführung als Vollbrücke;

Fig. 10: die Verschaltung der Widerstände als Ausführung als Vollbrücke mit zusätzlichem Spannungsteiler, und

Fig. 11: die Verschaltung der Widerstände mit einer Ausführung als Vollbrücke, mit allen Leitungswiderständen für die Auswertung durch Schalten.

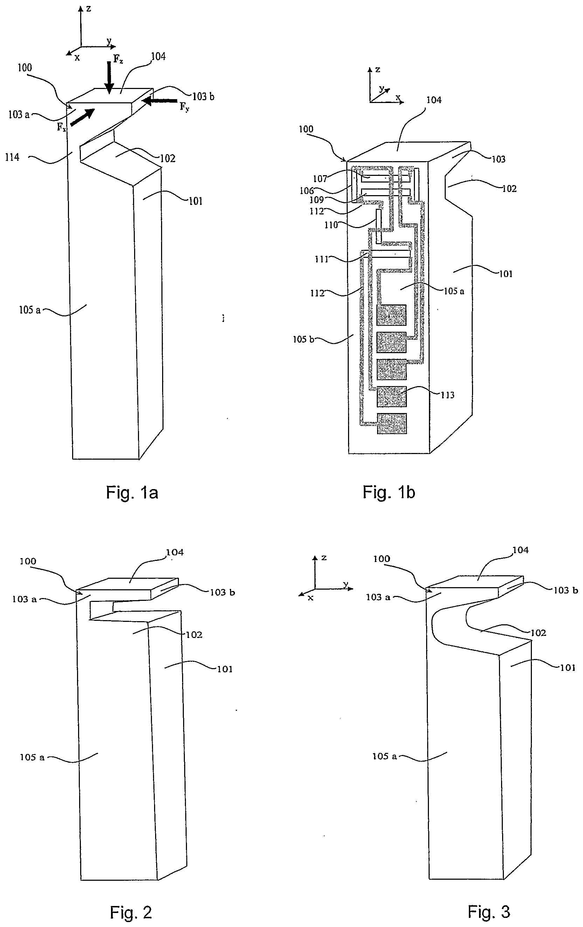

Das Messelement 100 nach **Fig. 1a** und b besteht aus einem Silizium-Grundkörper 101. In den Grundkörper 101 ist eine Aussparung 102 eingebracht. Eine über die Stirnfläche 104 eingeleitete Kraft $F_z$ bewirkt in dem Grundkörper 101 eine mechanische Druckspannung durch Stauchung des Grundkörpers 101. Aufgrund der unsymmetrischen Ausführung des Grundkörpers 101, hervorgerufen durch die Aussparung 102, tritt weiterhin ein Moment und somit eine Verbiegung des Grundkörpers 101 und der Biegeplatte 114 auf. Dieses Moment ist über der Biegeplatte 114 konstant und erzeugt im Grundkörper 101 und der Biegeplatte 114 mechanische Zugspannungen. Diese Zugspannungen überlagern die aus der Stauchung resultierende Druckspannung und überkompensieren diese, so dass eine resultierende Zugspannung in dem Grundkörper 101, vor allem aber in der Biegeplatte 114 entsteht. Deshalb können an der Oberseite des Grundkörpers 101 mit den eindotierten Widerständen $R_1$ bis $R_6$ mechanische Zugspannungen gemessen werden. Durch die Widerstandsänderung der Einzelwiderstände und über Brückendiagonalspannung kann die Kraftkomponente $F_z$ gemessen werden.

Bei einem seitlichen Kraftangriff $F_x$ ändert sich der Widerstand der Widerstände $R_5$ und $R_6$. Aufgrund der seitlichen Verbiegung des Messelementes 101 und der Platte 114 durch die Kraft $F_x$ wird der Widerstand $R_4$ einer Druckspannung und der Widerstand $R_2$ einer Zugspannung ausgesetzt. Dadurch sinkt der Widerstandswert von Widerstand $R_4$, und der Widerstandswert von Widerstand $R_2$ steigt. Somit ändert sich auch die Brückendiagonalspannung bei Belastung des Messelementes 100 mit einer Kraft $F_x$. Bei Kenntnis der Differenz der Widerstandsänderung der Widerstände $R_2$ und $R_4$ kann die Kraftkomponente $F_x$ bestimmt werden. Die Widerstandsänderung der Widerstände $R_3$ und $R_1$ bleibt bei einer Belastung des Messelementes 100 mit einer Kraft $F_z$ klein. Zur Messung der Einzelwiderstandsänderung wird eine Methode vorgeschlagen, so dass neben der axialen Kräfte $F_z$ auch seitliche Kräfte $F_x$ und $F_y$ gemessen werden können.

Wird über die radiale Stirnfläche 103 b in Fig. 1a eine Kraft $F_y$ in das Messelement 100 eingeprägt, entsteht eine weitere seitliche Verbiegung des Grundkörpers 100. Es entsteht ein Moment, dessen Betrag mit der Entfernung von der Krafteinleitungsstelle linear zunimmt. Hierdurch entstehen mechanische Zugspannungen, die mit den Widerständen $R_1$ bis $R_6$ gemessen werden können. Aufgrund des nicht konstanten Momentenverlaufs entlang der axialen Länge des Messelementes erfahren die Widerstände unterschiedliche Widerstandsänderungen. Durch Messung des mechanischen Spannungszustandes an mindestens zwei auf der Länge des Grundkörpers 101 verteilten Punkten kann der Momentenverlauf ermittelt werden und somit die Kraft $F_y$ bestimmt werden. Hierzu kann beispielsweise die Differenz der Widerstandsänderung der Widerstände $R_1$ und $R_3$ dienen. Eine höhere Messgenauigkeit kann erzielt werden, wenn die Messwiderstände in axialer Richtung möglichst weit voneinander im Abstand $\Delta x$ entfernt sind. Hierzu sind zusätzlich zu den Brückenwiderständen $R_1$ bis $R_4$ die Widerstände $R_5$ und $R_6$ eingebracht, die hier beispielsweise zu einem Spannungsteiler verschaltet und nicht zwangsweise in der Nähe des Einschnittes 102 lokalisiert sind. In optimaler Weise sind die Widerstände $R_5$ und $R_6$ an dem Ort angebracht, an dem sich die an dem sich Zug- und Druckspannungen die durch die Kraft $F_z$ entstehen, gerade kompensieren. Alternative Widerstandsanordnungen sind möglich.

In der **Fig. 2** ist die Aussparung 102 im Grundkörper 101 durch ein Verfahren mit einem sehr hohen Aspektverhältnis erzeugt, beispielsweise durch einen Trockenätzschritt von der Scheibenrückseite. Durch das hohe Aspektverhältnis der steilen Ätzkante der Aussparung 102 ist es möglich, die Aussparung 102 sehr nah an der Kopfplatte 104 zur platzieren um somit mit dem Messelement sehr empfindlich axiale Kräfte $F_z$ zu messen, aber eine geringe Empfindlichkeit für seitliche Kräfte $F_x$ und $F_y$ zu erzielen.

In einer weiteren Ausführungsform nach **Fig. 3** ist der Einschnitt des Grundkörpers durch Sägen von der Grundkörperrückseite erzeugt. Sägen ist für die Herstellung des Messelementes ein sehr günstiges Verfahren, da Sägen in der Regel zur Vereinzelung der strukturierten Messelemente aus der Scheibe eingesetzt wird. Weiterhin kann eine nahezu senkrechte Kante der Aussparung 102 erzeugt werden. Deshalb kann die Aussparung 102 näher an die Kopfplatte des Grundkörpers 101 eingebracht werden. Dies steigert die Funktionsfähigkeit des Messelementes, da seitliche Momente einen geringeren Einfluss auf das Messergebnis haben. Weiterhin entfallen bei der Strukturierung des Einschnittes mehrere Prozessschritte, da eine Strukturierung einer Ätzmaske auf der Scheibenrückseite entfallen kann. Dies sind im Speziellen mehrere Reinigungsschritte, das Aufbringen und Strukturieren der Ätzmaske, das Ätzen usw.. Das Verfahren kann mit bekannten Wafersägen durchgeführt werden. Zur Erhöhung der Stabilität des Messelementes kann nachfolgend ein kurzer chemischer Ätzschritt zur Verringerung von Mikrorissen erfolgen, wozu keine Maskierung der Rückseite erfolgen muss und auch nicht die hohen Anforderungen an die Genauigkeit bestehen wie bei bekannten, oben erwähnten Strukturätzschritten..

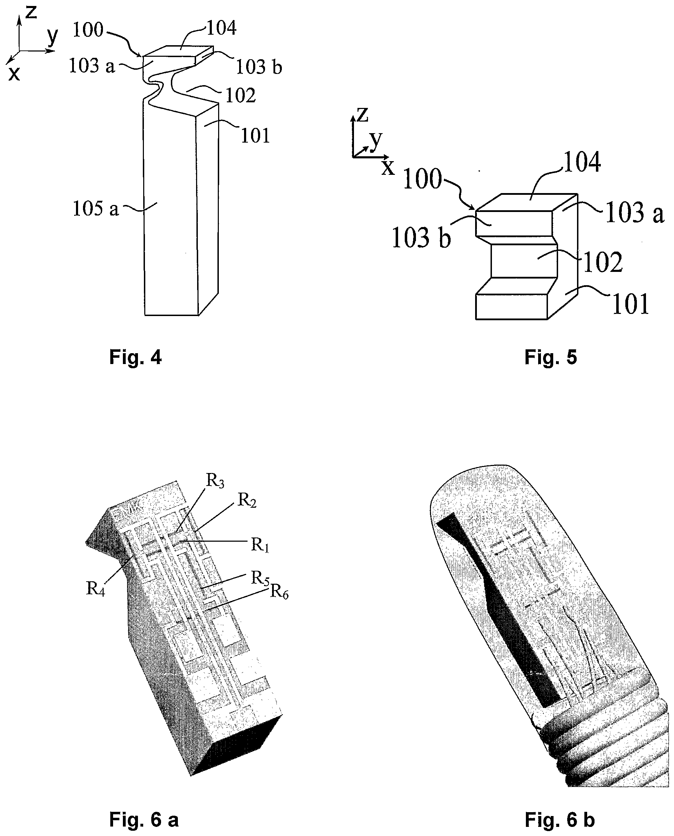

In einer weiteren Ausführungsform nach **Fig. 4** wird die Aussparung 102 zur Erzeugung der Asymmetrie des Grundkörpers nicht nur durch einen geraden Einschnitt auf der Scheibenrückseite erzeugt, sondern beispielsweise durch Bohren oder spezielle Ätzlöcher von der Scheibenrückseite oder von der Scheibenvorderseite. Es entsteht eine höhere Asymmetrie des Grundkörpers 101. Dadurch wird der Messeffekt weiter gesteigert.

Eine Möglichkeit, funktionsfähige Prototypen zu erzeugen, entsteht durch die Ausführungsform nach **Fig. 5** durch die Umarbeitung von in der Druckmesstechnik standardmäßig eingesetzten Messelementen. Hier wird durch einen Ätzprozess eine Abdünnung des Grundkörpers unter den Messwiderständen erzielt. Diese Messelemente weisen naturgemäß immer eine stabile Randstruktur auf. Durch Entfernen dieser Randbereiche kann diese Struktur in eine erfindungsgemäße Struktur überführt werden. Es können Standard-Drucksensoren mit geraden oder schrägen Seitenwänden verwendet werden. Hierzu ist der vorhandene Träger abzutrennen bzw. aufzutrennen oder direkt Messelemente ohne Träger zu verwenden. Durch Abtrennen oder Auftrennen von zwei parallelen Seitenwänden ist eine Struktur nach Fig. 5 erzeugbar. Das Abtrennen der Seitenflächen kann durch Sägen, Schleifen, Ätzen oder ein weiteres mikrotechnisches Bearbeitungsverfahren erfolgen. Diese Bearbeitung kann am einzelnen Element durchgeführt werden, wozu dieses mit der Biegeplatte auf einem Träger befestigt wird, um die empfindliche Biegeplatte vor Beschädigung zu schützen. Zur Befestigung kann beispielsweise ein Cyanacrylatkleber verwendet werden, der nach der Bearbeitung des Messelementes eine Ablösung, beispielsweise durch Aceton, zulässt. Die Seitenflächen bis zur Biegeplatte können auch durch ein Schleifverfahren beispielsweise mit Körnungen um 1000 entfernt werden. Für eine Serienfertigung ist eine Fertigung im Nutzen möglich, wozu Standard-Sägeverfahren verwendet werden können.

Eine CAD-Zeichnung eines erfindungsgemäßen Sensors, der für eine Integration in einen Führungsdraht mit 360 $\mu$m Durchmesser optimiert ist, zeigt **Fig. 6 a**.

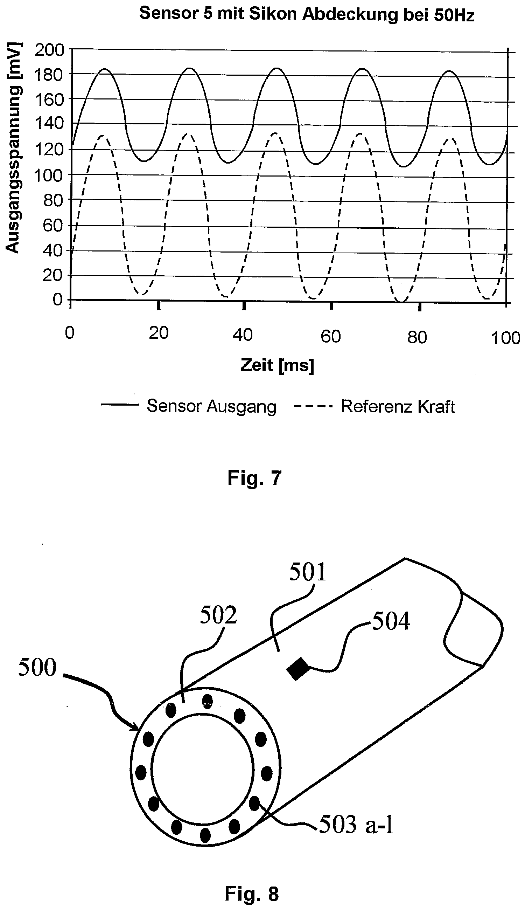

In **Fig. 6 b** ist ein in die Spitze eines Führungsdrahtes integrierter, elektrisch kontaktierter Sensor dargestellt. Die Gehäusung erfolgt hier durch einen Verguss mit einem relativ weichen, biokompatiblen Material, beispielsweise Silikon oder Polyurethan. Die hohe Steifigkeit des Messelementes im Vergleich zum Gehäusungswerkstoff gewährleistet einen geringen Einfluss der Gehäusung auf das Messsignal. Dies wird durch Messergebnisse eines gehäusten Prototyps nach Fig. 7 belegt.

**Fig. 7** zeigt Messergebnisse eines erfindungsgemäßen Sensors nach der Gehäusung mit einer Silikon-Vergussmasse. Dargestellt ist das Ausgangssignal des Sensors bei Anregung mit einer Kraft $F_z$ in axialer Richtung. Zu erkennen ist der reproduzierbare Krafteinfluss.

In **Fig. 8** ist eine Möglichkeit dargestellt, die Sensoren auch in Katheterschläuche zu integrieren. Es ist ein Schnitt durch die Katheterspitze dargestellt. Die Sensoren sind in den Katheter, beispielsweise durch Umspritzen bei der Herstellung des Katheters, integriert. Durch die hohe Miniaturisierung der Sensoren bleibt die distale Öffnung des Katheters voll erhalten. Die erfindungsgemäßen Kraftsensoren sind vorzugsweise am distalen Ende der lang gestreckten Einrichtung ein- oder angebracht, oder aber an einer beliebigen Stelle an oder in der lang gestreckten Einrichtung.

Aufgrund der einfacheren Herstellung werden die Widerstände in der Regel auf der Oberseite des Messelementes, und nicht in Vertiefungen in der Siliziumscheibe eingebracht, wie es auch im Folgenden angenommen werden soll. Eine Steigerung der Messempfindlichkeit kann bei dem erfindungsgemäßen Sensor aber erreicht werden, wenn die Widerstände auf der Rückseite der Biegeplatte in dem Einschnitt, beispielsweise durch Strukturierung mit Dickschicht-Resist, eingebracht werden. Dadurch bildet sich bei Kraftbelastung mit der Kraft $F_z$ durch die Biegung des Grundkörpers auf der Rückseite der Biegeplatte eine positive Druckspannung aus, die sich zu der positiven Druckspannung der Druckwirkung der Kraft $F_z$ addiert. Somit unterstützen sich beide

Es ist auch möglich, eine Auswerteelektronik direkt auf oder an dem Messelement anzubringen. Vorteile lassen sich beispielsweise erzielen, indem durch eine Vorverstärkung eine erhöhte Signalspannung erzeugt wird. Dadurch steigt das Signal- zu Rauschleistungsverhältnis (SNR) und somit auch die Auflösung. Durch codierte oder modulierte Übertragung der Signale oder durch spannungsproportionale Stromwandlung können bei integrierter Elektronik die Signale mit geringerem Einfluss der Zuleitung übertragen werden. Durch die Integration von Konstantspannungsquellen kann eine Konstantspannungsspeisung realisiert werden, so dass die Auflösung für seitliche Kraftkomponenten erhöht wird, ohne dass der Leitungswiderstand einen merkenswerten Einfluss auf das Messergebnis ausübt. Weiterhin ist es durch eine integrierte Elektronik möglich, elektrische Differenzspannungen an den Widerständen direkt zu messen und somit die Richtung und den Betrag von Kräften sehr genau zu bestimmen. Durch eine integrierte Elektronik kann eine Potentialanpassung der Signale auf dem Chip erfolgen und elektrische Differenzspannungen auf eine einzelne Masseleitung bezogen werden. Hierdurch können elektrische Leitungen eingespart werden. Dadurch sinkt der Montageaufwand und somit die Herstellungskosten der gesamten Einrichtung. Der Platzbedarf der Leitungen wird weiterhin verringert, wodurch eine weitere Miniaturisierung des Durchmessers der lang gestreckten Einrichtung möglich wird. Durch die Modulation der Signale in einer im Messelement integrierten Primärelektronik ist es möglich, die Leitungszahl auf zwei, und im Speziellen auf eine Leitung zu reduzieren. Durch Integration einer drahtlosen Sendeeinheit ist auch eine drahtlose Übertragung von Energie und Signal möglich.

Schließlich betrifft die Erfindung eine Methode zur Bestimmung des Betrags der auf die lang gestreckte Einrichtung wirkenden Kraft. Weitere Ausführungsformen dieser Methode ermöglichen im Besonderen die Bestimmung der Kraft nach Betrag und Richtung.

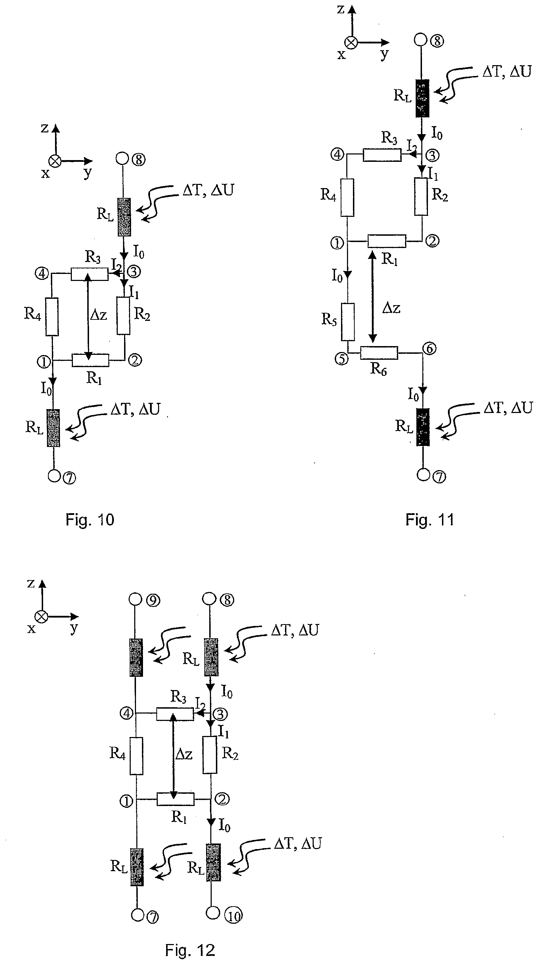

Im Folgenden soll eine Methode beschrieben werden, mit dem es möglich ist, durch Verwendung einer Wheatstone-Brückenschaltung nach **Fig. 9-11** in Verbindung mit dem erfindungsgemäßen Sensor drei unabhängige Kraftkomponenten - $F_x$, $F_y$ und $F_z$ - zu messen. In einer Ausführungsform nach **Fig. 10** wird ein zusätzlicher Spannungsteiler verwendet, um die verbleibende Messunsicherheit zur Unterscheidung der Kräfte aus x- und z-Richtung weiter zu verringern.

In einer ersten Ausführungsform Methode zur Bestimmung der Kraftkomponenten wird durch Strom- und Spannungsmessung zwischen den kontaktierbaren Punkten 2, 4, 5, 7 und 8 in Fig. 10 auf die Widerstandswerte $R_1$ bis $R_6$ geschlossen, wobei die Energieversorgung kontinuierlich über fest gewählte Anschlüsse erfolgt. Dies ermöglicht die kontinuierliche Bestimmung des anliegenden Kraftvektors.

In einer zweiten Ausführungsform der Anwendung des Sensors werden die Strom- bzw. Spannungsversorgung sowie die Signalleitungen des Messelementes durch eine Elektronik abwechselnd zwischen den Punkten 7-10 in **Fig. 11** in verschiedenen Kombinationen gewechselt bzw. die Signalleitung zeitweise gegen Masse geschaltet. Hierdurch können die Widerstandswerte besonders genau bestimmt werden. Die Erfassung des Kraftvektors wird dadurch zeitdiskret und eine Anpassung des Gesamtsystems umfassend Sensor, Energie- und Signalleitungen und Elektronik muss auf die geforderte Frequenzauflösung der Anwendung angepasst sein, um eine quasi-kontinuierliche Darstellung des Kraftvektors zu gewährleisten.

Die dem Verfahren zugrunde liegenden mathematischen Zusammenhänge sind von der Art der Energiespeisung der verwendeten Messbrücke abhängig. Aufgrund der großen Länge der im Allgemeinen sehr dünnen Zuleitungsdrähte wird aufgrund des hieraus resultierenden hohen Grundwiderstandes der Leitung und der damit verbundenen thermischen Widerstandsänderung während der Anwendung die Stromspeisung der Widerstände bevorzugt. Deshalb wird für die Erläuterung des Verfahrens die Konstantstromspeisung angenommen. Eine Speisung mit konstanter Spannung ist ebenso möglich, aber auch Verfahren bei denen die Messwiderstände mit einer zeitlich veränderlichen Energieversorgung gespeist werden, beispielsweise mit einem sinus- oder rechteckförmigen Signal- und Energiefluss, sind anwendbar. Die Zusammenhänge können dann von einem Fachkundigen durch einfache Berechnungen angepasst werden.

Zur Beschreibung der ersten Ausführungsform der Anwendung des erfindungsgemäßen Kraftsensors ist in **Fig. 9** schematisch der elektrische Aufbau bei Verschaltung der Messwiderstände zu einer Wheatstone-Vollbrücke angeführt. Zusätzlich ist ein Widerstand der Zuleitung aufgenommen, der durch äußere Temperatureinflüsse $\Delta T$ verändert wird. Weiterhin werden über die Leitungen elektromagnetische Störungen eingekoppelt. Diese sind mit $\Delta U$ bezeichnet. Die Messung der Ausgangsspannung erfolgt im Normalfall hochohmig, weshalb Leitungswiderstände im ersten Fall für die Ausgangsspannung vernachlässigt werden können und nicht dargestellt sind.

Im Folgenden wird aufgezeigt, wie mit Hilfe der elektrischen Spannungen an den in Fig. 9 dargestellten Widerstandsanordnungen unterschiedliche Kraftkomponenten gemessen werden können. Bei vorgegebener Stromstärke $I_0$ und gleichen Grundwiderständen $R_0$ ergeben sich die Ströme in den Brückenzweigen zu

$$I_1 = \frac{R_1 + \Delta R_1 + R_2 + \Delta R_2}{R_1 + \Delta R_1 + R_2 + \Delta R_2 + R_3 + \Delta R_3 + R_4 + \Delta R_4} \cdot I_0 = \frac{1}{2} \cdot \frac{\left(1 + \frac{\Delta R_1 + \Delta R_2}{2 \cdot R_0}\right)}{\left(1 + \frac{\Delta R_1 + \Delta R_2 + \Delta R_3 + \Delta R_4}{4 \cdot R_0}\right)} \cdot I_0$$

(Gl. 1)

Hierbei ist die Widerstandsänderung über den piezoresistiven Effekt mit den mechanischen Spannungen verknüpft. Es gilt für die gezeigten Messelemente vereinfacht

$$\frac{\Delta R_i}{R_0} = \pi_{l\ddot{a}ngs} \cdot T_{l\ddot{a}ngs} + \pi_{quer} \cdot T_{quer}$$

(Gl. 2)

wobei $T_{l\ddot{a}ngs}$ und $T_{quer}$ die mechanischen Spannungen längs und quer zur Widerstandausrichtung bezeichnen. $\pi_{l\ddot{a}ngs}$ und $\pi_{quer}$ sind die zugeordneten piezoresistiven Koeffizienten.

Analog zu $I_1$ lässt sich $I_2$ berechnen, oder es gilt auch einfach

$$I_2 = I_0 - I_1$$

(Gl. 3)

In einer Messbrücke nach Fig. 10 lassen sich mit diesen Strömen die Spannungen zwischen allen vier externen Anschlüssen der Brückenwiderstände bestimmen. Für die Brückendiagonalspannung an den Punkten 2 und 4 ergibt sich unter Vernachlässigung der quadratischen Anteile der auf den Grundwiderstand bezogenen kleinen Widerstandsänderungen der Zusammenhang

$$U_{2,4} = U_2 - U_4 = R_0 \left( 1 + \frac{\Delta R_2}{R_0} \right) \cdot I_1 - R_0 \left( 1 + \frac{\Delta R_4}{R_0} \right) \cdot I_2$$

$$\approx \frac{\frac{1}{4} \left( \frac{-\Delta R_1 + \Delta R_2 - \Delta R_3 + \Delta R_4}{R_0} \right)}{\left( 1 + \frac{\Delta R_1 + \Delta R_2 + \Delta R_3 + \Delta R_4}{4 \cdot R_0} \right)} \cdot R_0 \cdot I_0$$

(Gl. 4)

Die elektrische Spannung $U_{2,4}$ zwischen den Punkten 2 und 4 ist ein Maß für die mechanische Spannung in axialer Messelementrichtung, die sich vor allem durch die Kräfte $F_z$ und $F_y$ einstellt. Ist der Anteil der mechanischen Spannung aus dem Anteil von $F_y$ bekannt, dann kann auch der Anteil in $F_z$ und damit $F_z$ selbst ermittelt werden.

Weiterhin sind die Spannungen zwischen den Punkten 4 und 8 sowie zwischen den Punkten 2 und 7 zugänglich. Sie ergeben sich in folgender Weise:

$$U_{4,8} = R_3 \cdot I_2 + R_L \cdot I_0$$

$$\approx \left( \frac{\frac{1}{2} \cdot R_0 \cdot \left( 1 + \frac{2 \cdot \Delta R_3 + \Delta R_1 + \Delta R_2}{2 \cdot R_0} \right)}{\left( 1 + \frac{\Delta R_1 + \Delta R_2 + \Delta R_3 + \Delta R_4}{4 \cdot R_0} \right)} + R_L \right) \cdot I_0$$

(Gl. 5)

und

$$U_{2,7} = R_1 \cdot I_1 + R_L \cdot I_0$$

$$\approx \left( \frac{\frac{1}{2} \cdot R_0 \cdot \left( 1 + \frac{2 \cdot \Delta R_1 + \Delta R_3 + \Delta R_4}{2 \cdot R_0} \right)}{\left( 1 + \frac{\Delta R_1 + \Delta R_2 + \Delta R_3 + \Delta R_4}{4 \cdot R_0} \right)} + R_L \right) \cdot I_0$$

(Gl. 6)

Durch Differenzbildung der beiden Spannungen $U_{4,8}$ und $U_{2,7}$ in einer Elektronik kann der Offsetanteil beider Spannungen, der durch den Grundwiderstand $R_0$ und dem symmetrischen Leitungswiderstand $R_L$ entsteht, sowie eine elektromagnetische Störeinkopplung beseitigt werden. Es ergibt sich dadurch vorwiegend eine Differenz der Widerstandsänderungen von $R_1$ und $R_3$. Diese Differenzspannung ist ein Maß für das anliegende Moment um die y-Achse und somit ein Maß für die Kraft $F_y$, die hieraus bestimmt werden kann.

Damit ist die Kraft $F_y$ bekannt und das aus der Brückendiagonalspannung erhaltene Signal kann um den Anteil der Wirkung von $F_y$ verringert werden, so dass sich vorwiegend die Wirkung von $F_z$ ergibt und somit auch $F_z$ bekannt ist.

Ähnliche Gleichungen lassen sich für die quer angeordneten Widerstände formulieren:

$$U_{4,7} = R_4 \cdot I_2 + R_L \cdot I_0$$

$$\approx \left( \frac{1}{2} \cdot \frac{R_0 \cdot \left( 1 + \frac{2 \cdot \Delta R_4 + \Delta R_1 + \Delta R_2}{2 \cdot R_0} \right)}{\left( 1 + \frac{\Delta R_1 + \Delta R_2 + \Delta R_3 + \Delta R_4}{4 \cdot R_0} \right)} + R_L \right) \cdot I_0$$

(Gl. 7)

und

$$U_{2,8} = R_2 \cdot I_1 + R_L \cdot I_0$$

$$\approx \left( \frac{1}{2} \cdot \frac{R_0 \cdot \left( 1 + \frac{2 \cdot \Delta R_2 + \Delta R_3 + \Delta R_4}{2 \cdot R_0} \right)}{\left( 1 + \frac{\Delta R_1 + \Delta R_2 + \Delta R_3 + \Delta R_4}{4 \cdot R_0} \right)} + R_L \right) \cdot I_0$$

(Gl. 8)

Durch Differenzbildung der beiden Spannungen $U_{4,7}$ und $U_{2,8}$ in einer Elektronik kann wiederum der Offsetanteil beider Spannungen, der durch den Grundwiderstand $R_0$ und dem symmetrischen Leitungswiderstand $R_L$ entsteht, beseitigt werden. Es ist leicht erkennbar, dass bei eine gegensätzlichen Änderung der Widerstände $R_2$ und $R_4$, wie es bei einer Belastung des Messelementes mit einer Kraft $F_x$ entsteht, der Unterschied der Spannungen $U_{4,7}$ und $U_{2,8}$ wächst, wodurch die Kraft $F_x$ bestimmt werden kann.

Die drei Spannungen $U_{4,8}$, $U_{4,2}$ und $U_{2,7}$ bilden durch Einsetzen der piezoresistiven Verkopplungsgleichung drei unabhängige Gleichungen, in welche über die Sensorgeometrie die drei Kräfte $F_x$, $F_y$, und $F_z$ einfließen. Diese Gleichungen lassen sich außer durch analytische Berechnungen auch durch FEM-Simulationen unter Berücksichtigung des piezoresistiven Effektes und der elektrischen Verkopplung ermitteln, oder am realen Sensor durch Messen bestimmen. Da drei unabhängige elektrische Spannungen gemessen werden, ist das lineare Gleichungssystem durch Addition und Multiplikation der Spannungen so lösbar, dass $F_x$, $F_y$, und $F_z$ einzeln bestimmt werden können. Somit sind die drei Kraftkomponenten in Richtung und Amplitude messbar. Eine Extraktion der einzelnen Kraftkomponenten $F_x$, $F_y$ und $F_z$ kann in einer praktischen Umsetzung somit beispielsweise durch analoge Addierer und Verstärker oder aber auch digital erfolgen.

Die beschriebene Messmethode zur Messung mehrerer Kraftkomponenten ist mit einer Wheatstone-Brücke ohne Veränderungen und ohne die Verwendung zusätzlicher Leitungen möglich. Die Auflösung für die Hauptkomponente $F_z$, die durch die Brückendiagonalspannung $U_{2,4}$ gegeben ist, wird nicht verringert. Es ist darauf zu achten, dass die Widerstände $R_1$ und $R_3$ in dem hier vorliegenden speziellen Fall in einem entsprechenden Abstand $\Delta z$ angeordnet werden, um eine genügend große Differenz der durch die Kraftkomponente $F_x$ hervorgerufenen mechanischen Normalspannungen zu erfassen.

Die Auflösung für Kräfte aus y-Richtung kann erhöht werden, indem eine zusätzliche Widerstandsanordnung im Abstand $\Delta z$ nach Fig.11 auf dem Messelement angebracht wird. In Fig. 11 ist dies beispielsweise ein zusätzlicher Spannungsteiler aus piezoresistiven Widerständen, wie er am realen Messelement durch die Widerstände $R_5$ und $R_6$ in Fig. 1b dargestellt sind. Die Kraftkomponente Fy lässt sich dann aufgrund des größeren Abstandes $\Delta z$ und der höheren Empfindlichkeit des Spannungsteilers genauer auflösen. Hierzu kann beispielsweise die Differenz der elektrischen Spannung an den Punkten 4 und 5 in Fig. 11 dienen, aber auch andere Kombinationen der elektrischen Spannungen sind möglich.

Ähnliche Zusammenhänge lassen sich für Spannungsspeisung der Brücke finden, wobei zur Ermittlung der relevanten Gleichungen einfach die Spannungsteilerregel angewendet wird. Es sind dann höhere Übertragungsfaktoren für die seitlichen Kraftkomponenten $F_y$ und $F_x$ zu erwarten, aber auch eine Abhängigkeit der Brückendiagonalspannung von dem Leitungswiderstand.

Zusätzliche Informationen über die Widerstandsänderung der Einzelwiderstände lassen sich durch Messung des Innenwiderstandes der Brückenschaltung ableiten. Hierzu können an den externen Anschlüssen der Signalleitungen bekannte Widerstände angebracht werden und somit ein Stromfluss über die Signalleitungen bewusst zugelassen werden. Dieser Strom ist abhängig von allen vier Widerständen und den externen, zur Brücke parallelen Widerständen. Es kann eine Auswerteschaltung mit konstanten oder zeitlich veränderbaren externen Widerstandswerten realisiert werden. Anstatt externer Widerstände können auch Stromquellen in den Signalleitungen zur Belastung der Brücke verwendet werden, um über das Potenzial der Brückendiagonalspannung die Innenwiderstände zu bestimmen.

Unter Zuhilfenahme weiterer Randbedingungen, beispielsweise der Annahme eines konstanten Brückengesamtwiderstands unter mechanischer Belastung, lassen sich weitere Informationen über die Widerstandsänderung ableiten.

In einer weiteren Ausführungsform der Anwendung des Kraftsensors ist es auch möglich, die Leitungen der Brückenschaltung nach Fig. 9 durch eine externe Elektronik in einem zeitlichen Ablauf nur einzeln anzuschließen bzw. die Energieleitungen und Signalleitungen im Betrieb periodisch oder stochastisch zu vertauschen. Hierdurch können unterschiedliche Empfindlichkeiten für unterschiedliche Kraftrichtungen erzielt werden. Dann müssen auch die Leitungswiderstände der Signalleitungen berücksichtigt werden, da sie zeitweise Energieleitungen darstellen und Strom führen. Das vollständige Ersatzschaltbild ist in Fig. 11 dargestellt. Die Funktionsweise ist einfach. Beispielsweise kann über die Anschlüsse an den Punkten 7 und 8 in Fig. 12 ein Gesamt-Brückenwiderstand addiert mit dem Leitungswiderstand ermittelt werden. Durch eine zeitlich versetzte Widerstandsmessung zwischen den Punkten 10 und 9 kann ein weiterer Gesamtwiderstand ermittelt werden, der in der Regel nicht dem vorher gemessenen Widerstand zwischen den Punkten 7 und 8 entspricht. Beide Gesamtwiderstände lassen sich durch mathematische Funktionen der Brückenwiderstände beschreiben. Diese Gleichungen stellen unabhängige Linearkombinationen der Widerstandsänderungen und der Kraftkomponenten dar. Unter Zuhilfenahme weiterer Messungen in gleicher Weise zwischen den Punkten 7 bis 10 kann die Änderung der Einzelwiderstände berechnet werden kann.

Weiterhin ist es auch möglich, zwischen benachbarten Anschlüssen der Fig. 12 den Widerstand zu bestimmen, so dass der gemessene Widerstand der Parallelverschaltung des direkt anliegenden Widerstands mit den restlichen Widerständen, addiert mit den Leitungswiderständen entspricht.

Auch ist es mit einer Auswerteschaltung möglich, durch Schalten der Signalleitung gegen Masse, einen Kurzschluss der nun einseitig mit Masse verbundenen Widerstände zu bewirken. Dies kann beispielsweise in der Art erfolgen, dass an Punkt 8 nach Fig. 11 dauerhaft die Versorgungsspannung anliegt; an Punkt 7 dauerhaft Massepotential. Durch vorübergehendes Verbinden der Punkte 9 und 10 mit Massepotential und gleichzeitiger Messung des Stromflusses durch die Punkte 9 und 10 können die Widerstandswerte $R_2$ und $R_3$ bestimmt werden. Durch Öffnen der Schalter kann die normale Funktion der Brücke wieder hergestellt werden. Durch Messen der elektrischen Spannung beispielsweise zwischen den Punkten 7 und 10 sowie den Punkten 7 und 4 lassen sich dann die verbliebenen Widerstandswerte $R_1$ und $R_4$ bestimmen. Somit sind die Werte aller vier Widerstände bekannt. Zur Erhöhung der Messgenauigkeit können weitere Messungen, beispielsweise zwischen den Punkten 10 und 8 sowie zwischen den Punkten 9 und 8 vorgenommen und so der Einfluss des Leitungswiderstandes verringert werden, im Besonderen dann, wenn der Leitungswiderstand $R_L$ in allen Leitungen den selben Wert aufweist.

Besonders einfach und genau lässt sich die Widerstandsänderung von Einzeiwiderständen bestimmen, wenn eine geöffnete Brücke verwendet wird. Dies ist dann besonders vorteilhaft anzuwenden, wenn die Leitungswiderstände $R_L$ gering sind, da sonst aufgrund der Leitungswiderstände eine Unsymmetrie der Brückenverschaltung entsteht. Es ist bei der Ausführung als offene Brücke weiterhin mindestens eine weitere Zuleitung zu integrieren.

Einem Fachkundigen ist es leicht möglich, die Beziehungen zwischen elektrischen Spannungen und Strömen anzugeben und eine von vielfältigen Möglichkeiten zur Schaltungsstruktur zu entwerfen, mit der auf die Widerstandsänderung von Einzelwiderständen geschlossen werden kann. In dieser Art können in Kombination mit dem erfindungsgemäßen Kraftsensor unabhängige Kraftkomponenten gemessen und somit der Kraftvektor bestimmt werden.

## Bezugszeichenliste

| | |
|---|---|
| 100 | Kraftaufnehmer |
| 101 | Grundkörper |
| 102 | Einschnitt, z.B. hergestellt durch nasschemisches Ätzen |
| 103 a | radiale Stirnfläche in x-Richtung |
| 103 b | radiale Stirnfläche in y-Richtung |
| 104 | Stirnfläche / axiale Krafteinkopplung |
| 105 a | seitlicher Montagebereich |
| 105 b | elektrische Kontaktfläche und Montagebereich |
| $\alpha$ | Winkel, Seitenwand Einschnitt (102) zu senkrechter Fläche Einschnitt |
| $R_1$ | unterer Querwiderstand der Vollbrücke |

| | |
|---|---|
| $R_2$ | rechter Längswiderstand der Vollbrücke |
| $R_3$ | oberer Querwiderstand der Vollbrücke |
| $R_4$ | linker Längswiderstand der Vollbrücke |
| $R_5$ | Längswiderstand der zusätzlichen Halbbrücke |
| $R_6$ | Querwiderstand der zusätzlichen Halbbrücke |
| $R_L$ | Leitungswiderstand |
| 112 | Leiterbahn zur Verbindung der Widerstände |
| 113 | Elektrische Kontakte |
| 114 | Biegeplatte |
| 115 | Energie- und Signalleitungen |
| 116 | Führungsdrahtfeder |
| 500 | Katheterschlauch mit integrierten Kraftsensoren |
| 501 | Außenhülle des Katheters |
| 502 | distales Ende des Katheters |
| 503 | Kraftsensoren zur Aufnahme der Kräfte an der Katheterspitze |
| 504 | Kraftsensoren zur Aufnahme der Kräfte an einem beliebigen Ort des Katheters |
| F | Kraft allgemein |
| Fz | Komponente der Kraft in z-Richtung, eine frontal, axial zur lang gestreckten Einrichtung wirkende Kraftkomponente |
| $F_x$ | Komponente der Kraft in x-Richtung, eine seitlich, radial- zur lang gestreckten Einrichtung wirkende Kraftkomponente |
| $F_y$ | Komponente der Kraft in y-Richtung, eine seitlich, radial- zur lang gestreckten Einrichtung wirkende Kraftkomponente |

**Patentansprüche**

1. Kraftsensor zum Erfassen mindestens einer Kraftkomponente, insbesondere der in Längsrichtung einer lang gestreckten Einrichtung, insbesondere einer medizintechnische Einrichtung, wie einen Katheter oder Führungsdraht, wirkenden Kraft (F), umfassend einen Kraftaufnehmer (100) für die zu erfassende Kraft, bestehend aus einem Grundkörper (101) mit einer Stirnfläche (104), mindestens einem spannungs- und / oder dehnungsempfindlichen Widerstand ($R_1$ bis $R_6$), elektrische Anschlussflächen (113) des Sensors, und einen Anschluss 105 zum Anbringen des Sensors (100) an die lang gestreckte Einrichtung,
   **dadurch gekennzeichnet,**

   - **dass** der Grundkörper (101) einen Einschnitt (102) aufweist, der eine Asymmetrie des Grundkörpers bezüglich der Längsrichtung der lang gestreckten Einrichtung bewirkt, welche bei Kraftbelastung aus axialer Richtung ($F_z$) zu einer Verbiegung des Grundkörpers führt,
   - **dass** die Stirnfläche (104), einen Flächeninhalt aufweist, der wesentlich größer als die Querschnittsfläche des Grundkörpers (101) im Bereich des Einschnittes (102), aber nicht wesentlich kleiner als der Querschnitt des restlichen Grundkörpers ist
   - und **dass** die elektrischen Anschlussflächen 113 in einer nicht senkrechten Weise zur axialen Richtung der lang gestreckten Einrichtung angebracht sind.

2. Kraftsensor nach Anspruch 1, **dadurch gekennzeichnet, dass** er neben einer in Längsrichtung der lang gestreckten Einrichtung wirkende Kraft $F_z$ auch mindestens eine seitlich angreifende Kraftkomponente misst.

3. Kraftsensor nach Anspruch 1, **dadurch gekennzeichnet, dass** der Winkel $\alpha$ des Einschnittes (102) zwischen 30° und 150°, bevorzugt aber 90° beträgt, wobei der Einschnitt (102) von mehr als einer Seite erzeugt wird, und so eine große Asymmetrie bezüglich der Längsachse des Grundkörpers entsteht.

4. Kraftsensor nach Anspruch 1, **dadurch gekennzeichnet,**
   **dass** der Grundkörper (101) aus Silizium besteht und die Widerstände monolithisch integriert sind, oder dass der Grundkörper aus einem anderen Material besteht und zur Widerstandsmessung Dehnungsmessstreifen oder andere Hilfsmittel zur Dehnungs- oder Spannungsmessung angewendet werden, oder auf einem monokristallinen oder nicht monokristallinen Grundkörper anders geartete dehnungs- oder spannungsempfindliche Bauteile angebracht werden, oder dass der Grundkörper (101) aus verschiedenen Teilen des gleichen oder verschiedenen Materialien zusammengesetzt ist.

5. Kraftsensor nach Anspruch 1, **dadurch gekennzeichnet, dass** die Stirnfläche (104) plan oder rund oder beliebig ausgestaltet ist, oder mit einer Spitze in axialer Richtung versehen ist.

6. Kraftsensor nach Anspruch 1, **dadurch gekennzeichnet, dass** die Widerstände auf der Plattenober- oder auf der Plattenunterseite oder an anderer Stelle am Grundkörper angebracht sind, und dass mindestens ein Widerstand für jede zu messende Kraftkomponente integriert ist.

7. Kraftsensor nach Anspruch 6, **dadurch gekennzeichnet, dass** mindestens ein weiterer Widerstand oder eine Widerstandanordnung angebracht ist, so dass die Kraftkomponenten $F_z$ und $F_x$ aus einer Differenz der Widerstandsänderungen bestimmt werden können.

8. Kraftsensor nach Anspruch 6, **dadurch gekennzeichnet, dass** mindestens zwei Brückenwiderstände in einem vergrößerten Abstand angebracht sind, so dass aus dem Unterschied der Widerstandsänderungen die Wirkungen der seitlichen Kraftkomponenten $F_z$ und $F_x$ mit hoher Genauigkeit separiert werden können, wobei z. B. die zusätzliche Widerstandanordnung an dem Ort angebracht ist, an dem für die Kraftkomponente $F_z$ mindestens ein Widerstand oder eine Widerstandsanordnung ein minimales Messsignal zur Folge hat.

9. Kraftsensor nach Anspruch 6, **dadurch gekennzeichnet, dass** die Widerstände wenigstens teilweise zu Wheatstonschen-Brückenschaltungen verschaltet sind und diese offen oder geschlossen ausgeführt ist.

10. Kraftsensor nach Anspruch 1, **dadurch gekennzeichnet, dass** auf dem Messelement eine Elektronik zur Signalverarbeitung und / oder Energiespeisung und / oder Datenübertragung integriert ist.

11. Verfahren zur Verwendung eines Kraftsensors nach mindestens einem der Ansprüche 1 bis 10 zur Bestimmung des Kraftvektors mit Betrag und Richtung unter Verwendung einer Wheatstonschen-Brückenanordnung,
**dadurch gekennzeichnet,**
**dass** neben der Brückendiagonalspannung auch die Änderung der Einzelwiderstände bestimmt wird.

12. Verfahren nach Anspruch 11, **dadurch gekennzeichnet, dass** zur Verbesserung des Messergebnisses des Kraftsensors der Brückengesammtwiderstand gemessen wird und in die Auswertung der mechanischen Spannung einfließt.

13. Verfahren nach Anspruch 11, **dadurch gekennzeichnet, dass** zur Bestimmung der Kraftkomponenten neben der Brückendiagonalspannung weitere elektrische Differenzspannungen zwischen den zugänglichen Kontaktpunkten der Brückenschaltung und/oder die Leitungsströme zur Bestimmung des Kraftvektors verwendet werden und die Bestimmung des Kraftvektors kontinuierlich erfolgt.

14. Verfahren nach Anspruch 11, **dadurch gekennzeichnet, dass** zur Bestimmung der Kraftkomponenten der Widerstand von Einzelwiderständen durch zeitlich veränderliche Beschaltung / Kontaktierung der Widerständer erfolgt, und/oder dass diese zeitliche veränderliche Beschaltung / Kontaktierung der Widerstände periodisch oder zufällig und automatisch erfolgt, und/oder das die unterschiedliche Beschaltung der Widerstände durch schalten in einer externen Sekundärelektrode oder durch auf dem Messelement integrierte Schalter oder Modulatoren erfolgt, und/oder dass speziell die Leitungen zur Energieversorgung und die Signalleitungen zeitabhängig getauscht bzw. frei konfiguriert werden können.

15. Verfahren nach Anspruch 11, **dadurch gekennzeichnet, dass** zur Bestimmung der Kraftkomponenten die Daten und/oder die Energie moduliert oder multiplext oder anders codiert leitungslos oder leitungsgebunden übertragen werden, und/oder dass die Widerstände mit einem Strom oder einer Spannung mit konstanter Amplitude oder bestimmter Frequenz gespeist werden, und/oder dass die Signalverarbeitung analog und / oder digital extern erfolgt oder diese optional auf einem Chip integriert ist.

**Claims**

1. Force sensor for detecting at least one force component, in particular the force (F) acting in the longitudinal direction of an elongate device, in particular a medical device such as a catheter or guide wire, comprising a force receiver (100) for the force to be detected, consisting of a base body (101) having an end face (104) and at least one strain-sensitive and/or expansion-sensitive resistor ($R_1$ to $R_6$), electrical connection faces (113) of the sensor, and a connection (105) for attaching the sensor (100) to the elongate device,
**characterised in that**

- the base body (101) comprises an incision (102) which brings about an asymmetry of the base body about the longitudinal direction of the elongate device which leads to the base body deforming when force is applied from the axial direction ($F_z$),
- the end face (104) has an area which is much greater than the cross-sectional area of the base body (101) in the region of the incision (102), but not substantially smaller than the cross section of the rest of the base body,
- and the electrical connection faces (113) are attached non-perpendicular to the axial direction of the elongate device.

2. Force sensor according to claim 1, **characterised in that** in addition to a force $F_z$ acting in the longitudinal direction of the elongate device it also measures at least one laterally acting force component.

3. Force sensor according to claim 1, **characterised in that** the angle $\alpha$ of the incision (102) is between 30° and 150°, but preferably 90°, the incision (102) being produced from more than one face, resulting in a major asymmetry about the longitudinal axis of the base body.

4. Force sensor according to claim 1, **characterised in that** the base body (101) consists of silicon and the resistors are integrated in a single piece, or **in that** the base body consists of a different material and strain gauge strips or other expansion measurement or strain measurement means are used for resistance measurement, or expansion-sensitive or strain-sensitive components of different types are attached to a monocrystalline or non-monocrystalline base body, or **in that** the base body (101) is composed of various parts of the same or different materials.

5. Force sensor according to claim 1, **characterised in that** the end face (104) is planar or round or of any desired configuration or is provided with an apex in the axial direction.

6. Force sensor according to claim 1, **characterised in that** the resistors are attached to the upper or lower plate face or at another position on the base body, and **in that** at least one resistor is integrated for each force component to be measured.

7. Force sensor according to claim 6, **characterised in that** at least one further resistor or one resistor arrangement is attached, in such a way that the force components $F_z$ and $F_x$ can be determined from a difference between the changes in resistance.

8. Force sensor according to claim 6, **characterised in that** at least two bridge resistors are attached at an increased distance, in such a way that the effects of the lateral force components $F_z$ and $F_x$ can be separated out from the difference in the changes in resistance to a high precision, for example the additional resistor arrangement being attached at the location at which at least one resistor or one resistor arrangement results in a minimum measurement signal for the force component $F_z$.

9. Force sensor according to claim 6, **characterised in that** the resistors are at least in part connected to form Wheatstone bridge circuits, and these are formed open or closed.

10. Force sensor according to claim 1, **characterised in that** electronics for signal processing and/or power supply and/or data transmission are integrated onto the measurement element.

11. Method for using a force sensor according to at least one of claims 1 to 10 for determining the magnitude and direction of the force vector using a Wheatstone bridge arrangement,
**characterised in that**
in addition to the bridge diagonal voltage the change in the individual resistors is also determined.

12. Method according to claim 11, **characterised in that** to improve the measurement result of the force sensor the total bridge resistance is measured and taken into account in the evaluation of the mechanical strain.

13. Method according to claim 11, **characterised in that**, to determine the force components, in addition to the bridge diagonal voltage further electrical differential voltages between the accessible contact points of the bridge circuit and/or the line currents are used to determine the force vector, and the force vector is determined continuously.

**14.** Method according to claim 11, **characterised in that**, to determine the force components, the resistance of individual resistors takes place by connecting/contacting the resistors at different times, and/or **in that** the resistors are automatically connected/contacted at different times periodically or at random, and/or **in that** the resistors are connected differently by switching within an external secondary electrode or by way of switches or modulators integrated on the measurement element, and/or **in that** the lines for power supply and the signal lines can be replaced or freely configured specially in a time-dependent manner.

**15.** Method according to claim 11, **characterised in that**, to determine the force components, the data and/or power are transmitted in a modulated or multiplexed or otherwise coded form, in a wireless or wired manner, and/or **in that** the resistors are supplied with a current or voltage of a constant amplitude or a particular frequency, and/or **in that** the signal processing takes place externally in an analogue and/or digital manner or is optionally integrated onto a chip.

**Revendications**

**1.** Capteur de force destiné à détecter au moins une composante de force, notamment la force (F) agissant dans le sens longitudinal d'un dispositif oblong, notamment d'un dispositif médical, tel un cathéter ou un fil de guidage, comprenant un capteur de force (100) pour la force à détecter, constitué d'un corps de base (101) muni d'une surface frontale (104), d'au moins une résistance ($R_1$ à $R_6$) sensible à la tension et/ou à l'allongement, de surfaces de connexion électrique (113) du capteur et d'un raccordement (105) destiné à monter le capteur (100) sur le dispositif oblong,
**caractérisé**

- **en ce que** le corps de base (101) présente une encoche (102) qui provoque une asymétrie du corps de base par mont au sens longitudinal du dispositif oblong, laquelle, en cas de charge de force provenant de la direction axiale ($F_z$), entraîne une flexion du corps de base,
- **en ce que** la surface frontale (104) présente une aire de surface qui est essentiellement supérieure à la surface transversale du corps de base (101) au niveau de l'encoche (102) mais qui n'est pas essentiellement inférieure à la section transversale du corps de base restant
- et **en ce que** les surfaces de connexion électrique (113) sont montées de manière non perpendiculaire à la direction axiale du dispositif oblong.

**2.** Capteur de force selon la revendication 1, **caractérisé en ce que**, outre une force $F_z$ agissant dans le sens longitudinal du dispositif oblong, il mesure également au moins une composante de force ayant prise latéralement.

**3.** Capteur de force selon la revendication 1, **caractérisé en ce que** l'angle $\alpha$ de l'encoche (102) est compris entre 30° et 150°, **en ce qu'**il est cependant de préférence de 90°, l'encoche (102) étant générée par plus d'un côté, et une grande asymétrie par mont à l'axe longitudinal du corps de base étant ainsi créée.

**4.** Capteur de force selon la revendication 1, **caractérisé en ce que** le corps de base (101) est constitué de silice et en ce que les résistances sont monolithiquement intégrées, ou en ce que le corps de base est constitué d'un autre matériau et en ce que des jauges extensométriques sont utilisées pour la mesure de la résistance ou en ce que d'autres moyens auxiliaires sont utilisés pour la mesure de l'allongement ou de la tension, ou en ce que des composants d'un autre type, sensibles à l'allongement ou à la tension, sont montés sur un corps de base monocristallin ou non monocristallin, ou en ce que le corps de base (101) est assemblé à partir de différentes parties du même matériau ou de différents matériaux.

**5.** Capteur de force selon la revendication 1, **caractérisé en ce que** la surface frontale (104) est conçue de manière plane ou ronde ou de manière quelconque ou **en ce qu'**elle est munie d'une pointe en direction axiale.

**6.** Capteur de force selon la revendication 1, **caractérisé en ce que** les résistances sont montées sur le côté supérieur ou le côté inférieur de plaque ou à un autre endroit sur le corps de base, et **en ce qu'**au moins une résistance est intégrée pour chaque composante de force à mesurer.

**7.** Capteur de force selon la revendication 6, **caractérisé en ce qu'**au moins une résistance supplémentaire ou un agencement de résistances est monté, de sorte que les composantes de force $F_z$ et $F_x$ peuvent être déterminées à partir d'une différence des variations de résistance.

**8.** Capteur de force selon la revendication 6, **caractérisé en ce qu'**au moins deux résistances de pont sont montées avec un écart agrandi, de sorte qu'à partir de la différence des variations de résistance, les effets des composantes de force latérales $F_z$ et $F_x$ peuvent être séparés avec haute précision, dans lequel, par exemple, l'agencement de résistances supplémentaire est monté à l'endroit auquel au moins une résistance ou un agencement de résistances a pour conséquence un signal de mesure minimal pour les composantes de force $F_z$.

**9.** Capteur de force selon la revendication 6, **caractérisé en ce que** les résistances sont connectées au moins en partie en circuits en pont de Wheatstone et **en ce que** ceux-ci sont réalisés de manière ouverte ou fermée.

**10.** Capteur de force selon la revendication 1, **caractérisé en ce que** sur l'élément de mesure est intégré un équipement électronique destiné au traitement de signaux et/ou à l'alimentation d'énergie et/ou au transfert de données.

**11.** Procédé d'utilisation d'un capteur de force selon au moins l'une quelconque des revendications 1 à 10 pour déterminer le vecteur de force avec grandeur et direction, avec utilisation d'un agencement de pont de Wheatstone, **caractérisé en ce que**, outre la tension diagonale de pont, la variation de chacune des résistances est également déterminée.

**12.** Procédé selon la revendication 11, **caractérisé en ce que** pour améliorer le résultat de mesure du capteur de force, la résistance totale de pont est mesurée et prise en compte dans l'évaluation de la tension mécanique.

**13.** Procédé selon la revendication 11, **caractérisé en ce que** pour déterminer les composantes de force, outre la tension diagonale de pont, des tensions électriques différentielles supplémentaires entre les points de contact accessibles du circuit en pont et/ou les courants de ligne sont utilisées pour déterminer le vecteur de force et **en ce que** la détermination du vecteur de force est réalisée de manière continue.

**14.** Procédé selon la revendication 11, **caractérisé en ce que** pour déterminer les composantes de force, la résistance de résistances individuelles est réalisée par câblage / mise en contact temporellement modifiable des résistances, et/ou **en ce que** ce câblage / cette mise en contact temporellement modifiable des résistances est réalisé/e périodiquement ou de manière aléatoire et automatiquement, et/ou **en ce que** le différent câblage des résistances est réalisé par couplage dans une électrode secondaire externe ou par commutateur intégré sur l'élément de mesure ou par modulateurs intégrés sur l'élément de mesure, et/ou **en ce que** spécialement les lignes d'alimentation en énergie et les lignes de signalisation peuvent être échangées ou librement configurées en fonction du temps.

**15.** Procédé selon la revendication 11, **caractérisé en ce que** pour déterminer les composantes de force, les données et/ou l'énergie sont transférées de manière modulée ou multiplexée ou autrement codée, sans ligne ou de manière filaire, et/ou **en ce que** les résistances sont alimentées d'un courant ou d'une tension à amplitude constante ou fréquence déterminée, et/ou **en ce que** le traitement de signaux est réalisé de manière externe analogique et/ou numérique ou **en ce que** celui-ci est intégré dans une puce de manière optionnelle.

Fig. 1a

Fig. 1b

Fig. 2

Fig. 3

**Fig. 4**

**Fig. 5**

**Fig. 6 a**

**Fig. 6 b**

**Sensor 5 mit Sikon Abdeckung bei 50Hz**

—— Sensor Ausgang   - - - - Referenz Kraft

**Fig. 7**

**Fig. 8**

Fig. 10

Fig. 11

Fig. 12

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 3940696 A1
- DE 10303270 A1
- DE 10319081
- US 6221023 B1
- JP 06190050 A

**In der Beschreibung aufgeführte Nicht-Patentliteratur**

- **BECCAI L et al.** Silicon-based three axial Force Sensor for Prothetic Applications. Sensors and Microsystems. *Proccedings of the 7th Italien Conference,* 2002